# EUROPEAN PATENT APPLICATION

(11) **EP 2 293 133 A2**
(43) Date of publication of application: **09.03.2011**
(21) Application number: 10175577.5
(22) Date of filing: 07.09.2010
(51) Int. Cl.: G02B 23/26

(54) **Endoscope having optical fibers**

(30) Priority: 08.09.2009 JP 2009207048
(71) Applicant: FUJIFILM Corporation, Tokyo (JP)
(72) Inventor: Ozaki, Takao, Kanagawa (JP); Kuroda, Osamu, Kanagawa (JP); Nakamura, Takayuki, Kanagawa (JP); Iida, Takayuki, Kanagawa (JP)
(74) Representative: Klunker . Schmitt-Nilson . Hirsch

(57) **Abstract**

An endoscope for imaging of an object in a body cavity by receiving image light on an image sensor (56) is provided. In an elongated tube (9) for entry in the body cavity, an objective lens system (30) is incorporated for receiving the image light from the object. A fiber optic image guide (31) includes plural bundled optical fibers (52) and a distal tip (48), is inserted in the elongated tube, for transmitting the image light focused on the distal tip by the objective lens system. A piezoelectric actuator (35) is positioned on the distal tip, for displacing the distal tip periodically. The image sensor detects the image light from the fiber optic image guide for plural times in synchronism with the displacement for forming a synthesized image. An anchoring device (18, 86, 91) anchors the elongated tube partially on a wall of the body cavity, to stabilize the elongated tube relative to the image light.

## Description

### BACKGROUND OF THE INVENTION

### 1. Field of the Invention

The present invention relates to an endoscope having optical fibers. More particularly, the present invention relates to an endoscope having optical fibers in which an elongated tube can have a reduced diameter, and images of high image quality can be produced by removing instability in an orientation of a head assembly of the elongated tube.

### 2. Description Related to the Prior Art

An endoscope is an important medical instrument used in the field of the clinical medicine. Examples of the endoscope include primitive models such as a fiberscope or stomach camera, an electronic endoscope containing a CCD, and a capsule endoscope which is orally swallowed by a patient to retrieve an image.

In the field of endoscopic examination, extreme thinning of a tube for a reduced diameter to produce an ultra thin tube is desired very seriously for an elongated tube of the endoscope. Various ideas for the extreme thinning for a reduced diameter have been suggested for the purpose of imaging of various body parts in a narrow lumen, such as a pancreatic duct, bile duct, breast duct, terminal bronchioles, and the like.

The fiberscope is structurally suitable for the extreme thinning for a reduced diameter, because the image can be retrieved only by having a fiber optic image guide and an illuminating light guide. The fiber optic image guide transmits image light of the image of a body part or object of interest. The illuminating light guide applies light to the body part. However, cladding of an optical fiber bundle constituting the fiber optic image guide does not contribute to the transmission of the image light. There occurs a problem in that a pattern of mesh of a loss region due to the cladding appears locally within the image, and the image quality of the image will be low.

In view of this problem, U.S.P. No. 4,618,884 (corresponding to JP-A 60-053919) discloses the fiberscope. In a first embodiment of the document, a focusing lens system is disposed at a distal tip of the fiber optic image guide for focusing of image light on the distal tip. A piezoelectric actuator vibrates the focusing lens system to remove light component of a pattern of mesh from the image. The piezoelectric actuator vibrates the focusing lens system horizontally and vertically at a predetermined amount according to a pixel pitch of the pixels of the CCD or the optical fibers in the fiber optic image guide.

In a second embodiment of U.S.P. No. 4,618,884, the CCD is disposed at a distal end of the elongated tube without the fiber optic image guide. The focusing lens system in front of the CCD is vibrated in the same manner as its first embodiment. During the vibration, image light of the image is received on pixels of the CCD in a time division manner. Data are obtained, and written to a frame memory sequentially, to produce one frame of the image. Thus, high definition of the image can be obtained.

The focusing lens system has a larger diameter than the fiber optic image guide to ensure high brightness in the image. In U.S.P. No. 4, 618, 884, the focusing lens system is vibrated by the piezoelectric actuator. Even with the focusing lens system having the larger diameter than the fiber optic image guide, a further space is required for positioning a frame or retention mechanism for supporting the focusing lens system in a pivotally movable manner. The elongated tube must have a larger size in the radial direction. It follows that vibrating the focusing lens system with the piezoelectric actuator is inconsistent to the extreme thinning for a reduced diameter. A space required for positioning such a frame or retention mechanism is a serious problem in view of the extreme thinning for a reduced diameter of an order from tens of microns to a number of millimeters.

Although high definition of an image can be obtained from the second embodiment of U.S.P. No. 4,618,884, the extreme thinning for a reduced diameter is still impossible because the CCD is disposed in front of the elongated tube in addition to the focusing lens system.

Therefore, development of an endoscope system has been suggested in which a distal tip of a fiber optic image guide is displaced periodically by a piezoelectric actuator, and an obj ect is imaged for plural times in synchronism with the displacement. One synthesized image is created from plural output images and also in consideration of information of shift amounts. This is for meeting purposes of achieving an ultra thin tube and obtaining images of high quality.

In the suggested endoscope, a head assembly of the elongated tube may vibrate in a condition with the displacement of the distal tip of the fiber optic image guide as the head assembly is a free end in a body cavity. Image quality will be seriously low due to image shake. It may be conceivable to raise the weight of the head assembly for the purpose of reducing influence of the displacement. However, operability of the endoscope will be considerably low.

### SUMMARY OF THE INVENTION

In view of the foregoing problems, an object of the present invention is to provide an endoscope having optical fibers in which an elongated tube can have a reduced diameter, and images of high image quality can be produced by removing instability in an orientation of a head assembly of the elongated tube.

In order to achieve the above and other objects and advantages of this invention, an endoscope for imaging of an obj ect in a body cavity by receiving image light on an image sensor is provided. There is an elongated tube for entry in the body cavity. An objective lens system is incorporated in the elongated tube, for receiving the image light from the object. A fiber optic image guide includes a plurality of bundled optical fibers and a distal tip, is inserted in the elongated tube, for transmitting the image light focused on the distal tip by the objective lens system. A piezoelectric actuator is positioned on a periphery of the distal tip of the fiber optic image guide, for displacing the distal tip periodically, wherein the image sensor detects the image light from the fiber optic image guide for plural times in synchronism with the displacement for forming a synthesized image. An anchoring device anchors the elongated tube partially on a wall of the body cavity, to stabilize a position of the elongated tube relative to the image light incident thereon.

The anchoring device includes a resilient balloon, secured to a peripheral surface of the elongated tube removably in an air tight manner, for expanding by delivery of fluid with a pump to push the wall of the body cavity.

Furthermore, a fluid channel is formed through the elongated tube, for delivering the fluid between the balloon and the pump.

Furthermore, a sleeve device is disposed about the elongated tube. The anchoring device is positioned on a peripheral surface of the sleeve device.

In one preferred embodiment, the anchoring device includes a suction hole, formed in a peripheral surface of the elongated tube, for sucking the wall of the body cavity by generating a negative pressure with a suction pump.

Furthermore, a suction channel is formed through the elongated tube, for drawing air between the suction hole and the suction pump.

In another preferred embodiment, the anchoring device includes an ejection nozzle, formed in a peripheral surface of the elongated tube, for ejecting anchoring agent delivered by a delivery pump.

Furthermore, a delivery channel is formed through the elongated tube, for delivering the anchoring agent from the delivery pump to the ejection nozzle.

The anchoring device is actuated in response to a signal input with an input interface.

The input interface determines whether the distal tip should be displaced to generate a displacement signal. If the input interface generates the displacement signal, then the anchoring device is automatically actuated.

Consequently, images of high image quality can be produced, because the distal tip is surely anchored on a wall of a body cavity for stabilization.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an endoscope system;
Fig. 2 is a front elevation illustrating a head assembly of an endoscope of the endoscope system;
Fig. 3A is an explanatory view in a vertical section, illustrating the head assembly and a balloon to be assembled;
Fig. 3B is an explanatory view in a vertical section, illustrating the same as Fig. 3A but in an assembled state;
Fig. 4 is a vertical section illustrating the head assembly;
Fig. 5 is a perspective view illustrating a displacing device;
Fig. 6 is a front elevation illustrating a bundle of optical fibers of a fiber optic image guide;
Fig. 7 is a block diagram illustrating relevant elements in the endoscope system;
Fig. 8 is an explanatory view in a front elevation illustrating a relationship between an image transmitted by the core and a pixel of a CCD;
Fig. 9 is an explanatory view illustrating one example of displacement;
Fig. 10A is an explanatory view in a front elevation illustrating a two-dimensional path of a distal tip of one of the cores;
Fig. 10B is an explanatory view in a front elevation illustrating another two-dimensional path of the distal tip;
Fig. 11 is a block diagram illustrating relevant circuits for operation upon designating a composite imaging mode;
Fig. 12 is a timing chart illustrating a relationship between driving of the CCD, a piezoelectric control signal and an image synthesis signal;
Fig. 13 is a flow chart illustrating operation of the endoscope system;
Fig. 14 is an explanatory view illustrating one preferred embodiment for anchoring in which suction holes are formed in the head assembly;
Fig. 15 is an explanatory view illustrating another preferred embodiment for anchoring in which ejection nozzles are formed in the head assembly.

### DETAILED DESCRIPTION OF THE PREFERRED

### EMBODIMENT(S) OF THE PRESENT INVENTION

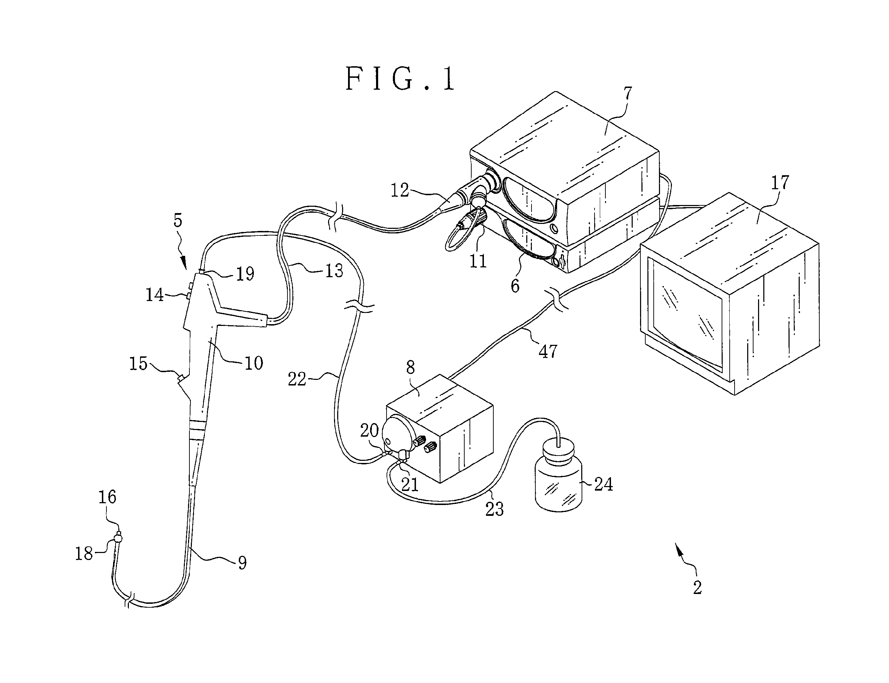

In Fig. 1, an endoscope system 2 includes an endoscope 5, a processing apparatus 6, a light source apparatus 7, and a pump apparatus 8. The endoscope 5 is used for imaging of various body parts in narrow lumens, for example, a pancreatic duct, bile duct, breast duct, terminal bronchioles, and the like. The endoscope 5 includes an elongated tube 9 or insertion tube, a handle 10, a first connector 11, a second connector 12 or coupler, and a universal cable 13. The elongated tube 9 is flexible and entered in a patient's body. The handle 10 is disposed at a proximal end of the elongated tube 9. The first connector 11 is plugged in the processing apparatus 6. The second connector 12 is plugged in the light source apparatus 7. The universal cable 13 extends from the handle 10 to the first connector 11 and the second connector 12.

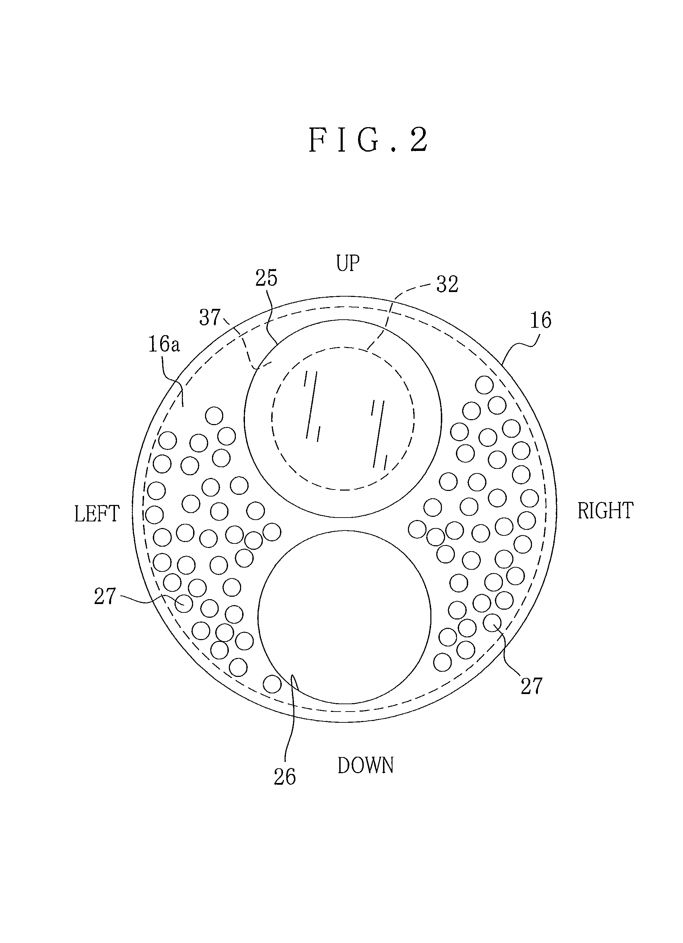

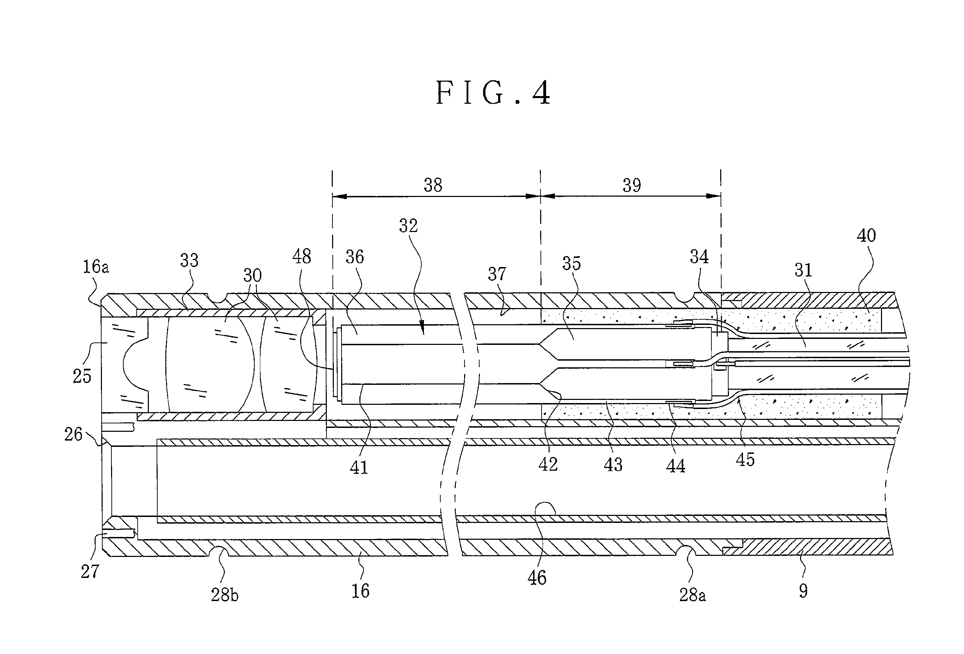

The elongated tube 9 has a thickness of 50 microns and outer diameter of 0.9 mm, and is formed from flexible material such as a Teflon (trade name), namely tetrafluoroethylene. A recording button 14 or release button among various buttons is disposed on the handle 10 for recording an endoscopic still image of a body part. An instrument opening 15 or forceps opening is formed on a side of the handle 10, and receives passage of an electrosurgical knife or other instruments for treatment. A head assembly 16 is disposed at a distal end of the elongated tube 9. An instrument opening 26 or forceps opening (See Fig. 2) is formed in the head assembly 16. A working channel 46 or forceps channel (See Fig. 4) is formed through the elongated tube 9, and extends from the instrument opening 15 to the instrument opening 26.

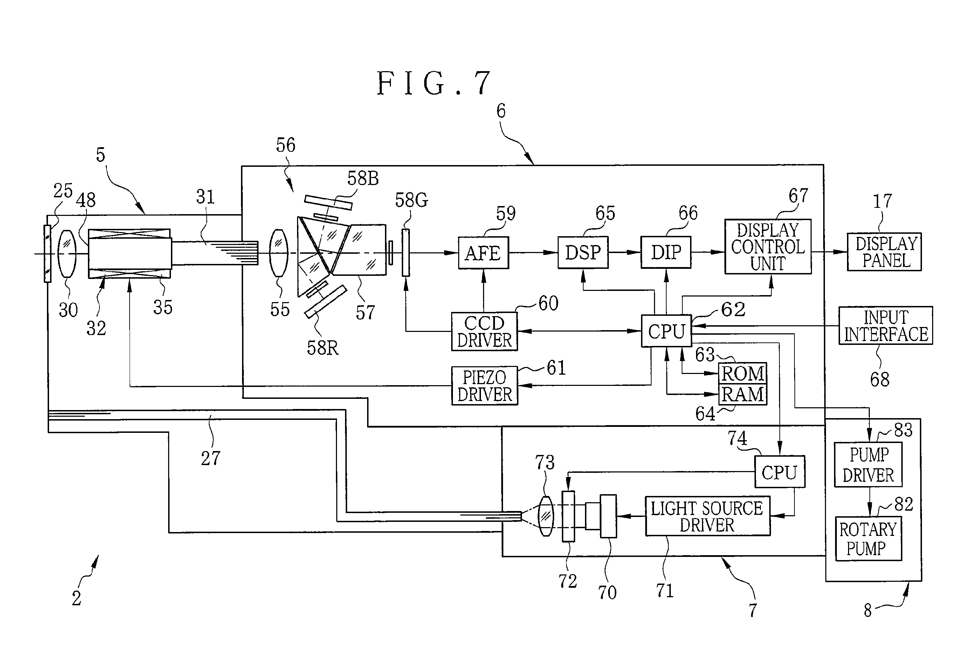

The processing apparatus 6 is connected with the light source apparatus 7 electrically, and controls operation of the constituents of the endoscope system 2. A connection cable 45 of Fig. 4 is inserted through the universal cable 13 and the elongated tube 9, and supplies the endoscope 5 with power from the processing apparatus 6. A displacing device 32 of Fig. 4 is also controlled by the processing apparatus 6. A fiber optic image guide 31 and a CCD group 58 are contained in the processing apparatus 6. The CCD group 58 includes CCDs 58B, 58G and 58R or image pickup devices. See Fig. 7. Image light of an image of a body part is transmitted by the fiber optic image guide 31, and received by the CCD group 58, which generates an image signal. The processing apparatus 6 processes the image signal in image processing, to create an image. A monitor display panel 17 is connected by use of a cable, and displays the image created by the processing apparatus 6.

The head assembly 16 has a wall constituted by a pipe of a stainless steel and has a thickness of 25 microns and an outer diameter of 0.8 mm. In Fig. 2, a distal end face 16a of the head assembly 16 is illustrated. An imaging window 25 is disposed in an upper portion of the distal end face 16a. The instrument opening 26 is open in the distal end face 16a and disposed under the imaging window 25. A plurality of light guide devices 27 or illumination fiber optics are contained in the head assembly 16. Ends of the light guide devices 27 are positioned beside the imaging window 25 and the instrument opening 26 without bundling and packed randomly in a tube lumen inside the head assembly 16.

The instrument opening 26 has an outer diameter of 0.34 mm and an inner bore of 0.3 mm, and is an exit opening of the working channel 46. See Fig. 4. An example of material of a wall of the working channel 46 is polyimide. An example of the light guide devices 27 is an optical fiber having a diameter of 50 microns. The light guide devices 27 are inserted through the elongated tube 9 and the universal cable 13, and have a proximal end located in the second connector 12 or coupler. Light is entered through the proximal end of the light guide devices 27, is transmitted, and is applied to an object of interest through a distal end of the light guide devices 27 positioned within the distal end face 16a.

For the light guide devices 27, a plurality of optical fibers without bundling are inserted in the elongated tube 9. Adhesive agent in a fluid form is supplied into the head assembly 16 for adhesion of the light guide devices 27. It is possible according to requirements to polish the surface of the distal end of the light guide devices 27 after the adhesion, or to dispose a lighting window in front of the distal end of the light guide devices 27 to cover the same. Also, a coating of phosphor or other materials may be applied to the lighting window to diffuse the light.

In Fig. 1, a balloon 18 as an anchoring device for stabilization is secured on a borderline between the elongated tube 9 and the head assembly 16. An example of material of the balloon 18 is latex rubber. The balloon 18 is entered in a body cavity in a compressed state of tightly sticking on the outer surface of the head assembly 16. To observe an object of interest, the balloon 18 is expanded by entry of water charged by the pump apparatus 8. The water is preferably degassed water free from dissolved gas after degassing. After the expansion, the balloon 18 is compressed by discharge of water by the pump apparatus 8.

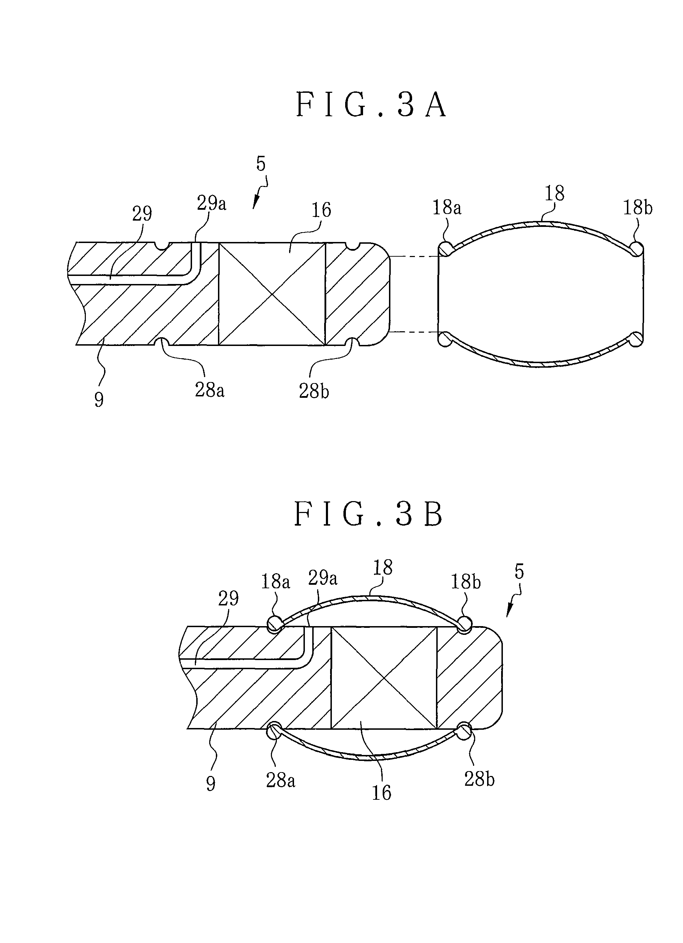

A fluid channel 29 is formed through the elongated tube 9 and the handle 10 for charge and discharge of water in the balloon 18. See Fig. 3. A first end of the fluid channel 29 is open in the head assembly 16. A connection opening 19 is formed in a proximal end of the handle 10. A second end of the fluid channel 29 is open with the connection opening 19.

The pump apparatus 8 includes two fluid ports 20 and 21. A fluid line 22 and an external fluid line 23 or flexible tubes are connected by coupling with respectively the fluid ports 20 and 21. A first end of the fluid line 22 is coupled with the fluid port 20. A second end of the fluid line 22 is coupled with the connection opening 19. A first end of the external fluid line 23 is coupled with the fluid port 21. There is a tank 24 with which a second end of the external fluid line 23 is coupled. Thus, a single path of flow is defined by the tank 24, the external fluid line 23, the pump apparatus 8, the fluid line 22 and the fluid channel 29 to the balloon 18.

A rotary pump 82 of Fig. 7 is installed in the pump apparatus 8 for generating a flow of fluid or water by rotating a rotor, and for selectively changing a flow direction of fluid by changing the rotational direction of the rotor. The pump apparatus 8 drives the rotary pump 82 to draw fluid through one of the fluid ports 20 and 21 and to deliver fluid through a remaining one of those.

When a composite imaging mode to be described later is selected, the pump apparatus 8 controls the rotary pump 82 to draw water through the fluid port 21 and deliver water through the fluid port 20, and introduces water from the tank 24 toward the balloon 18. Also, when a normal imaging mode is selected by changeover from the composite imaging mode, the pump apparatus 8 controls the rotary pump 82 to draw water through the fluid port 20 and deliver water through the fluid port 21, and returns water from the balloon 18 toward the tank 24.

In Fig. 3A, the balloon 18 has a shape with intermediate portions protruding from a cylindrical shape. Retaining rings 18a and 18b or end rings are formed with ends of the balloon 18 in a circular shape. A diameter of the retaining rings 18a and 18b is slightly smaller than that of the head assembly 16.

A pair of receiving grooves 28a and 28b are formed along the borderline defined between the elongated tube 9 and the head assembly 16. The receiving grooves 28a and 28b extend in a circular shape according to the retaining rings 18a and 18b of the balloon 18. Their form as viewed in a cross section is arcuate. An interval between the receiving grooves 28a and 28b is substantially equal to that between the retaining rings 18a and 18b. To secure the balloon 18, at first the retaining rings 18a and 18b are widened for the balloon 18 to receive insertion of the head assembly 16. The retaining rings 18a and 18b are fitted in the receiving grooves 28a and 28b with resiliency of the balloon 18. In Fig. 3B, the balloon 18 is secured firmly on the borderline between the elongated tube 9 and the head assembly 16 in an air tight manner. A fluid port 29a is formed between the receiving grooves 28a and 28b. An end of the fluid channel 29 appears at the fluid port 29a. Water is charged to or discharged from the balloon 18 through the fluid port 29a.

In Fig. 4, an objective lens system 30 is disposed behind the imaging window 25 together with the fiber optic image guide 31 and the displacing device 32. The displacing device 32 shifts the fiber optic image guide 31. A lens barrel 33 contains the objective lens system 30. A distal tip 48 for receiving light is positioned on a plane where image light of an image from an object is in focus. A diameter of the objective lens system 30 is 0.35 mm. An outer diameter of the lens barrel 33 is 0.4 mm. A length of the lens barrel 33 in the axial direction is 3.2 mm.

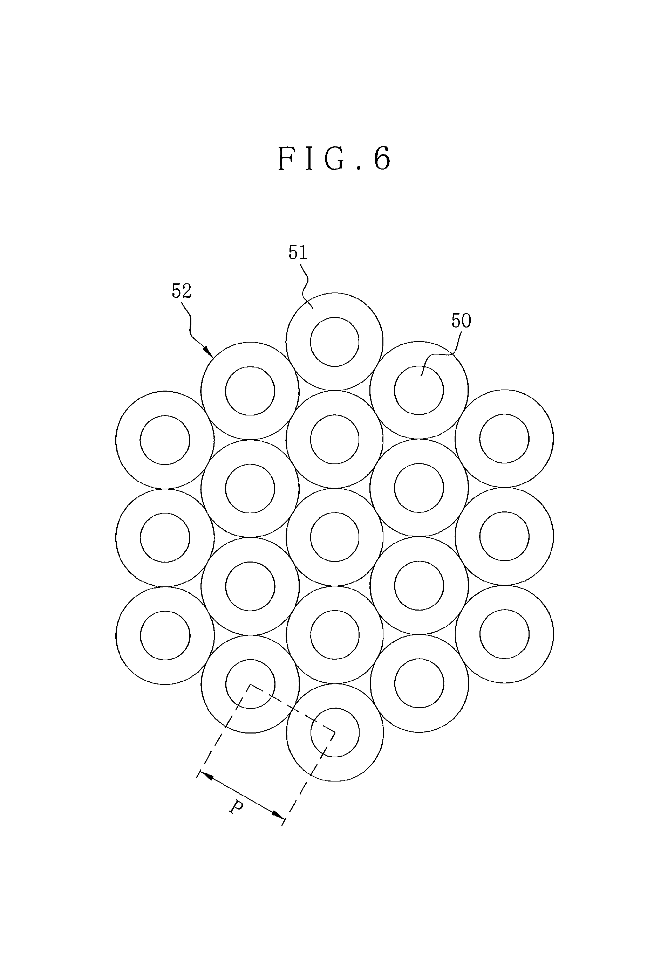

The fiber optic image guide 31 is a bundle of optical fibers with a diameter of 0.2 mm. See Fig. 6. The fiber optic image guide 31 extends through the elongated tube 9 and the universal cable 13, and has a proximal tip contained in the first connector 11. The fiber optic image guide 31 transmits image light of an object received from the objective lens system 30 through the distal tip 48 toward its proximal tip.

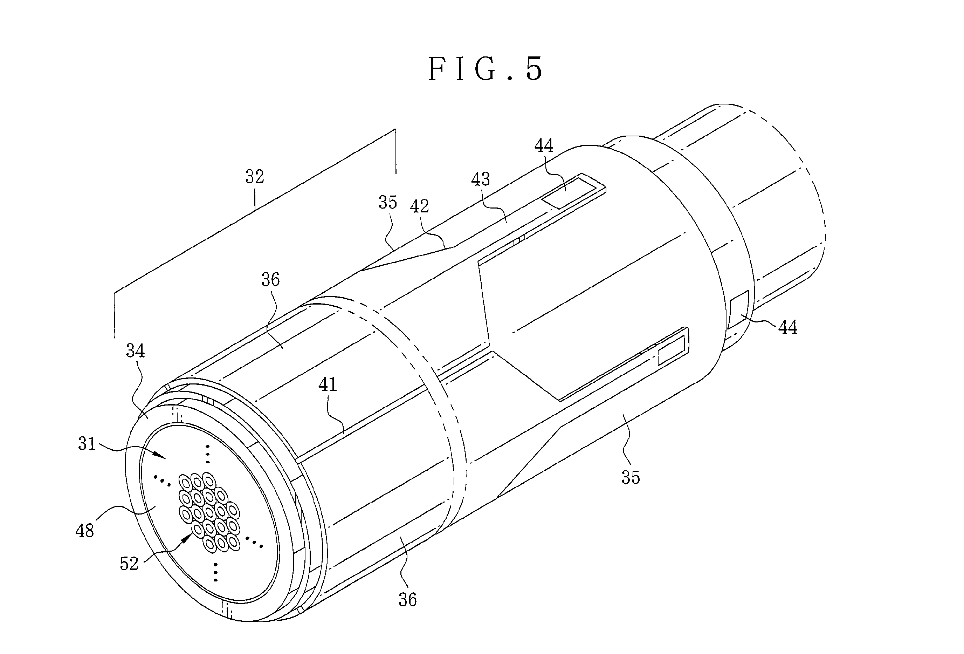

In Fig. 5, the displacing device 32 includes a support casing 34, a piezoelectric actuator material 35 and electrodes 36. The support casing 34 is a barrel or pipe of stainless steel, and has an outer diameter of 0.26 mm and an inner bore of 0.2 mm. The fiber optic image guide 31 is inserted in and fixed on the support casing 34. The piezoelectric actuator material 35 has a thickness of 15 microns, and is a coating applied to an outer surface of the support casing 34 in a cylindrical form. The electrodes 36 have a thickness of 5 microns, and are a coating about the piezoelectric actuator material 35.

The displacing device 32 is contained in a wall of the head assembly 16. A lumen 37 is defined between the outside of the displacing device 32 and the inside of the wall of the head assembly 16, and has a width of approximately 0.1 mm.

The displacing device 32 includes a shift mechanism 38 and a stationary section 39. The shift mechanism 38 is a portion of the displacing device 32 free from the wall of the head assembly 16 without fixing. The fiber optic image guide 31 is displaceable within the lumen 37 with respect to the stationary section 39. Adhesive agent 40 is used in the stationary section 39, and attaches the displacing device 32 to the inner wall of the head assembly 16. An area of the adhesive agent 40 extends from a proximal point of the displacing device 32 where the fiber optic image guide 31 appears to a point near to a distal end of the elongated tube 9. Lengths of the shift mechanism 38 and the stationary section 39 are respectively 4 mm and 1.9 mm in the axial direction. A length of filling of the adhesive agent 40 in the axial direction is 3.2 mm inclusive of the stationary section 39 and a distal portion of the elongated tube 9.

The electrodes 36 are arranged in the circumferential direction regularly at an angle of 90 degrees. The electrodes 36 are oriented with an inclination of 45 degrees relative to the vertical or horizontal direction of Fig. 2. Four grooves 41 are formed to extend in parallel with the axial direction, and define the electrodes 36 of two pairs. The electrodes 36 have a locally large width in the shift mechanism 38, as an interval between the electrodes 36 is only as great as the width of the grooves 41. In contrast, recesses 42 are defined with the electrodes 36 in the area of the stationary section 39, and extend in a symmetrical manner from the grooves 41. A narrow portion 43 of the electrodes 36 is defined by the recesses 42. The narrow portion 43 extends to the vicinity of the proximal end of the piezoelectric actuator material 35. The grooves 41 and the recesses 42 are formed by etching after applying a coating of the electrode material to the entire surface of the piezoelectric actuator material 35.

Pads 44 are disposed at proximal ends of the narrow portion 43. The connection cable 45 is connected with each of the pads 44. Also, the end of the support casing 34 has another one of the pads 44, with which the connection cable 45 is connected. Consequently, the support casing 34 operates as a common electrode for the piezoelectric actuator material 35.

The connection cable 45 has a cable diameter of 15 microns and an outer jacket diameter of 20 microns. The connection cable 45 is extended about the fiber optic image guide 31, inserted in the elongated tube 9 and the universal cable 13, and connected by the first connector 11 with the processing apparatus 6.

The two pairs of the electrodes 36 are supplied with voltages of opposite polarities with reference to a voltage applied to the support casing 34 as a common electrode. For example, let the support casing 34 have a potential of 0 V. An upper one of the electrodes 36 is supplied with +5 V. A lower one of the electrodes 36 is supplied with -5 V. The piezoelectric actuator material 35 under the electrodes 36 expands and contracts axially. In response to this, a portion of the shift mechanism 38 in front of the stationary section 39 displaces in the lumen 37 together with the distal tip 48 of the fiber optic image guide 31. It is possible to displace the shift mechanism 38 at a predetermined angle and amount by changing a combination of the electrodes 36 for powering and levels of the voltages.

In Fig. 6, the fiber optic image guide 31 has plural optical fibers 52, for example 6,000 fibers, bundled with extreme tightness in an equilateral hexagonal form. Each of the optical fibers 52 includes a core 50 and a cladding 51. A diameter of the core 50 is 3 microns. A diameter of the cladding 51 is 6 microns. A pitch P of arrangement of the optical fibers 52 is 6 microns.

In Fig. 7, the processing apparatus 6 includes an enlarging lens system 55 and a three-CCD assembly 56. The enlarging lens system 55 is opposed to the proximal tip of the fiber optic image guide 31 extending to the outside of the first connector 11. The enlarging lens system 55 enlarges the object image from the fiber optic image guide 31 with a suitable magnification, and directs its image light to the three-CCD assembly 56.

The three-CCD assembly 56 is an image sensor disposed behind the enlarging lens system 55. A color separation prism 57 is combined with the CCD group 58 to constitute the three-CCD assembly 56 as well-known in the art. The color separation prism 57 includes three prism blocks and two dichroic mirrors disposed on optical faces of the prism blocks. The color separation prism 57 separates image light of a body part from the enlarging lens system 55 into light components of red, blue and green colors, which are directed to the CCD group 58. The CCD group 58 outputs an image signal according to light amounts of the light components from the color separation prism 57. Note that a CMOS image sensor may be used instead of the CCD.

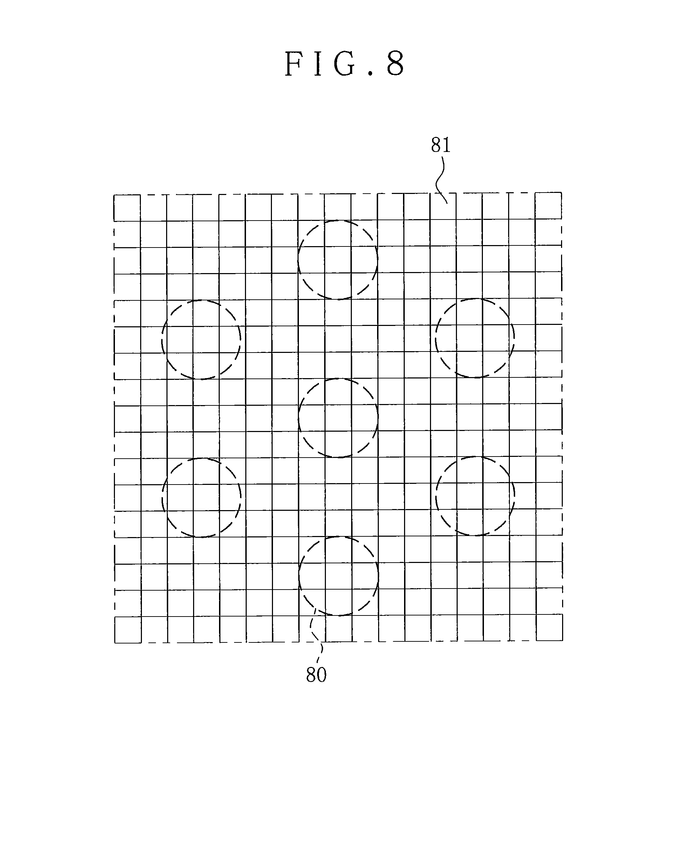

Image light of a part image 80 is transmitted by the core 50 of the fiber optic image guide 31. There are pixels 81 arranged on the image pickup surface of the CCD group 58. In Fig. 8, the part image 80 is viewed in a state projected to the image pickup surface of the CCD group 58. A center of the part image 80 substantially coincides with the center of a set of nine of the pixels 81. The proximal tip of the fiber optic image guide 31 is positioned relative to the color separation prism 57 and the CCD group 58 so as to correlate the part image 80 with the pixels 81 in the depicted state.

In Fig. 7, an analog front end 59 or AFE is supplied with the image signal from the CCD group 58. The analog front end 59 includes a correlated double sampling circuit or CDS circuit, an automatic gain control circuit or AGC circuit, and an A/D converter. The CDS circuit processes the image signal from the CCD group 58 in the correlated double sampling, and eliminates noise generated by the CCD group 58, such as reset noise, amplification noise and the like. The AGC circuit amplifies the image signal with a predetermined signal gain after noise elimination in the CDS circuit. The A/D converter converts the amplified image signal from the AGC circuit into a digital signal with a predetermined number of bits. A digital signal processor 65 or DSP has a frame memory (not shown) to which the digital form of the image signal from the A/D converter is written.

A CCD driver 60 generates drive pulses for the CCD group 58 and a sync pulse for the analog front end 59. The drive pulses include a vertical/horizontal scan pulse, electronic shutter pulse, reading pulse, reset pulse and the like. The CCD group 58 responds to the drive pulses from the CCD driver 60, takes an image, and outputs an image signal. Components included in the analog front end 59 are operated according to the sync pulse from the CCD driver 60. Note that the CCD driver 60 and the analog front end 59 are connected with the CCD 58G in the drawing, but also connected with the CCDs 58R and 58B.

A piezoelectric driver 61 is connected by the connection cable 45 with the electrodes 36 and the support casing 34. A CPU 62 controls the piezoelectric driver 61 to supply the piezoelectric actuator material 35 with voltage.

The CPU 62 controls the entirety of the processing apparatus 6. The CPU 62 is connected with various elements by a data bus (not shown), address bus, control lines and the like. A ROM 63 stores data (such as graphic data) and programs (operation system and application programs) for controlling the processing apparatus 6. The CPU 62 reads the program and data required for the purpose from the ROM 63. A RAM 64 is a working memory with which the CPU 62 performs tasks with data for operation by running the program. An input interface 68 is also associated with the CPU 62. The CPU 62 is supplied with information related to the examination by the input interface 68 or the LAN (local area network) or other networks, the information including a date and time of the examination, personal information of a patient, doctor's name, other text information, and the like. The CPU 62 writes the information to the RAM 64.

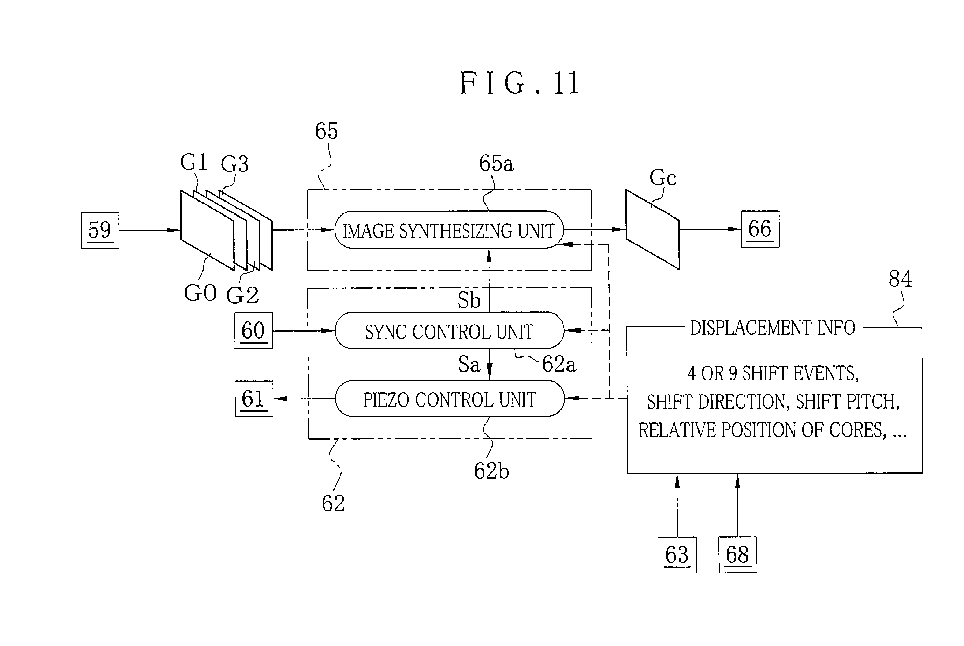

The digital signal processor 65 reads an image signal produced by the analog front end 59 from the frame memory. The digital signal processor 65 processes the image signal in processing of various functions, such as color separation, color interpolation, gain correction, white balance adjustment, gamma correction and the like, and produces an image of one frame. Also, the digital signal processor 65 has an image synthesizing unit 65a. See Fig. 11. When a composite imaging mode (to be described later) is selected, the image synthesizing unit 65a outputs one synthesized image of a high definition by combining plural images obtained in one two-dimensional shift sequence. To this end, plural frame memories are incorporated in the digital signal processor 65. A digital image processor 66 includes a frame memory (not shown), to which the image or synthesized image from the digital signal processor 65 is written.

The digital image processor 66 is controlled by the CPU 62 for image processing. The digital image processor 66 reads images from the frame memory after processing in the digital signal processor 65. Examples of functions of the image processing in the digital image processor 66 are electronic zooming, color enhancement, edge enhancement and the like. A display control unit 67 is supplied with data of the image processed by the digital image processor 66.

The display control unit 67 has a VRAM for storing the processed image from the digital image processor 66. The display control unit 67 receives graphic data read by the CPU 62 from the ROM 63 and the RAM 64. Examples of the graphic data include data of a mask for display of an active pixel area by masking an inactive area in the endoscopic image, text information such as an examination date, patient's name, and doctor's name, and data of graphical user interface (GUI), and the like. The display control unit 67 processes the image from the digital image processor 66 in various functions of display control, the functions including superimposition of the mask, the text information and the GUI, graphic processing of data for display on the display panel 17, and the like.

The display control unit 67 reads an image from the VRAM, and converts the image into a video signal suitable for display on the display panel 17, such as a component signal, composite signal or the like. Thus, the endoscopic image is displayed by the display panel 17.

The input interface 68 is a well-known input device, of which examples are an input panel on a housing of the processing apparatus 6, buttons on the handle 10 of the endoscope 5, mouse, keyboard, or the like. The CPU 62 operates relevant elements in the processing apparatus 6 in response to an input signal from the input interface 68.

The processing apparatus 6 also includes an image compression device, a media interface and a network interface. The image compression device compresses images in a format of compression, for example JPEG format. The media interface operates in response to an input from the recording button 14, and records the compressed images to a recording medium such as a CF card, MO (optical magnetic disk), CD-R and other removable media. The network interface transmits or receives various data by use of the LAN or other networks. Those are connected to the CPU 62 by a data bus or the like.

A light source 70 is incorporated in the light source apparatus 7. Examples of the light source 70 are a xenon lamp, white LED and the like which generate light of a broad band of the wavelength from red to blue, for example, with a wavelength of 480-750 nm. A light source driver 71 drives the light source 70. An aperture stop device 72 is disposed in front of the light source 70, and adjusts an amount of incident light. A condenser lens 73 condenses the light passed through the aperture stop device 72, and directs the light to the distal end of the light guide devices 27. A CPU 74 communicates with the CPU 62 of the processing apparatus 6, and controls the light source driver 71 and the aperture stop device 72.

A pump driver 83 is included in the pump apparatus 8 in combination with the rotary pump 82, and drives the rotary pump 82 for charge and discharge of water in the balloon 18. A cable 47 connects the pump driver 83 to the CPU 62 of the processing apparatus 6. See Fig. 1.

When the composite imaging mode is selected, the CPU 62 sends an expansion signal to the pump driver 83 to instruct expansion of the balloon 18. The pump driver 83 responsively drives the rotary pump 82 by controlling a direction and speed of rotation of its rotor, and expands the balloon 18 by charging water from the tank 24.

When the normal imaging mode is selected by changeover from the composite imaging mode, the CPU 62 sends a compression signal to the pump driver 83 for instructing compression of the balloon 18. The pump driver 83 responsively drives the rotary pump 82, and compresses the balloon 18 by discharging the water to the tank 24.

A flow meter is included in the pump apparatus 8 for measuring amounts of water drawn to flow by the rotary pump 82 according to the flow rate. The flow meter is connected with the pump driver 83, and outputs information of the amounts of water to the pump driver 83. The pump driver 83 responds to the information of the amounts from the flow meter, and controls the rotary pump 82 to adjust the amounts of the water at reference levels predetermined suitably. Note that the reference levels can be determined according to a feature of the object of interest, material of the balloon 18, its capacity and the like.

There are two imaging modes including a normal imaging mode without operating the displacing device 32 and a composite imaging mode in operation of the displacing device 32. In the composite imaging mode, the number of shift events is changeable between four and nine. The input interface 68 can be operated to change over the imaging modes and set the number of shift events.

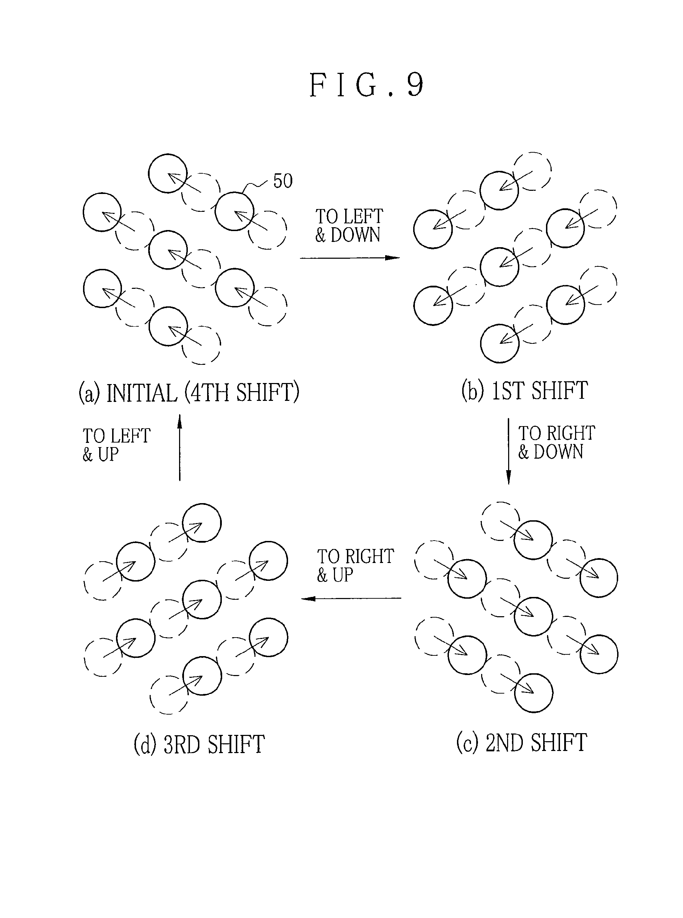

When the composite imaging mode is selected to set the four shift events, the piezoelectric driver 61 drives the shift mechanism 38 of the displacing device 32 to displace the distal tip 48 of the fiber optic image guide 31 as illustrated in Fig. 9. At first, the shift mechanism 38 displaces the distal tip 48 laterally from the initial position of (a) leftwards and downwards with an inclination of 30 degrees, and with an amount half as much as the pitch P of arrangement of the optical fibers 52. The shift mechanism 38 sets the distal tip 48 in a set position of (b) with a first shift event. Then the distal tip 48 is displaced at the same amount in the rightward and downward direction, and set in a set position of (c) with a second shift event. In sequence, the distal tip 48 is displaced at the same amount in the rightward and upward direction, and set in a set position of (d) with a third shift event. Then the distal tip 48 is displaced at the same amount in the leftward and upward direction, and set in an initial position of (a) with a fourth shift event by way of the initial position. The shift mechanism 38 is stopped in the set positions stepwise by the piezoelectric driver 61. Note that the solid line in the drawing indicates an actual position of the core 50 at the distal tip 48. The broken line indicates a previous position of the core 50 before the actual position.

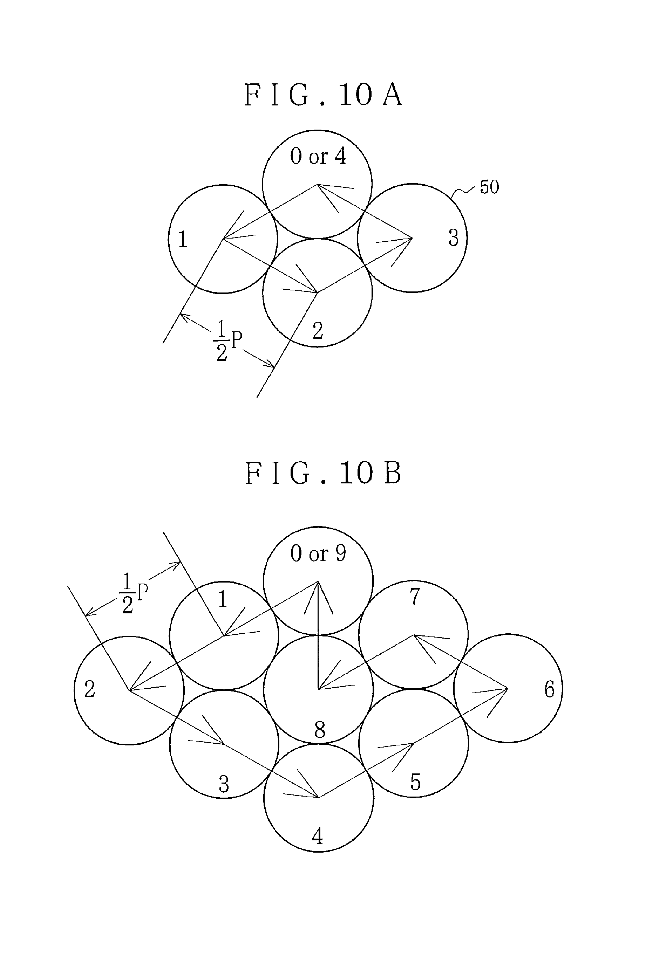

The core 50 in the distal tip 48 of the fiber optic image guide 31 repeatedly displaces in a composite sequence from the initial position of (a) to the set positions of (b), (c) and (d) then to the initial position of (a). The distal tip 48 shifts in a two-dimensional path of a polygonal shape of a rhombus of Fig. 10A to compensate for a loss region due to the cladding 51 in transmitting image light according to the initial position of (a).

Let a number of shift events be nine (9). In Fig. 10B, a two-dimensional path according to the nine shift events is illustrated. The number of shift events in each of the directions is one larger than that according to the mode of the four shift events. Note that a lateral direction from the seventh set position to the eighth set position is downward in contrast with the upward direction from the sixth set position to the seventh set position. A lateral direction from the eighth set position to the initial position or the ninth set position is upward with an angle of 90 degrees. In a manner similar to the mode of the four shift events, the two-dimensional path according to the nine shift events is in a shape to compensate for a loss region of the cladding 51 in transmitting image light according to the initial position. Furthermore, the distal tip 48 is displaced to the positions of the second, fourth and sixth set positions, which are the same as initial positions of three adjacent cores among the cores 50.

In Fig. 11, the composite imaging mode is designated. A sync control unit 62a and a piezoelectric control unit 62b are started in the CPU 62 of the processing apparatus 6. According to displacement information 84, the image synthesizing unit 65a of the digital signal processor 65 cooperates with the sync control unit 62a and the piezoelectric control unit 62b to perform various tasks.

The displacement information 84 is data related to shift events of the shift mechanism 38 of the displacing device 32. Examples of the data are a number of shift events, shift direction, shift pitch, relative positions of the pixels 81 of the CCD group 58 and the part image 80 transmitted through the core 50 of the fiber optic image guide 31 of Fig. 8. The data of the number of shift events is generated by the input interface 68. The ROM 63 stores basic datasets of the shift direction, shift pitch, relative positions of the pixels 81 and the part image 80. Any of those is read from the ROM 63 to the image synthesizing unit 65a, the sync control unit 62a and the piezoelectric control unit 62b.

The sync control unit 62a receives information of the drive pulses for the CCD group 58 from the CCD driver 60, and sends the piezoelectric control signal Sa to the piezoelectric control unit 62b and the image synthesis signal Sb to the image synthesizing unit 65a. The piezoelectric control unit 62b controls the piezoelectric driver 61 for fine displacement in synchronism with the piezoelectric control signal Sa. Similarly, the image synthesizing unit 65a performs a task of image synthesis in synchronism with the image synthesis signal Sb. Pixels of images G0, G1, G2 and G3 obtained from the set positions (in the mode of the four shift events) are mapped in accordance with the set positions, to create one synthesized image Gc.

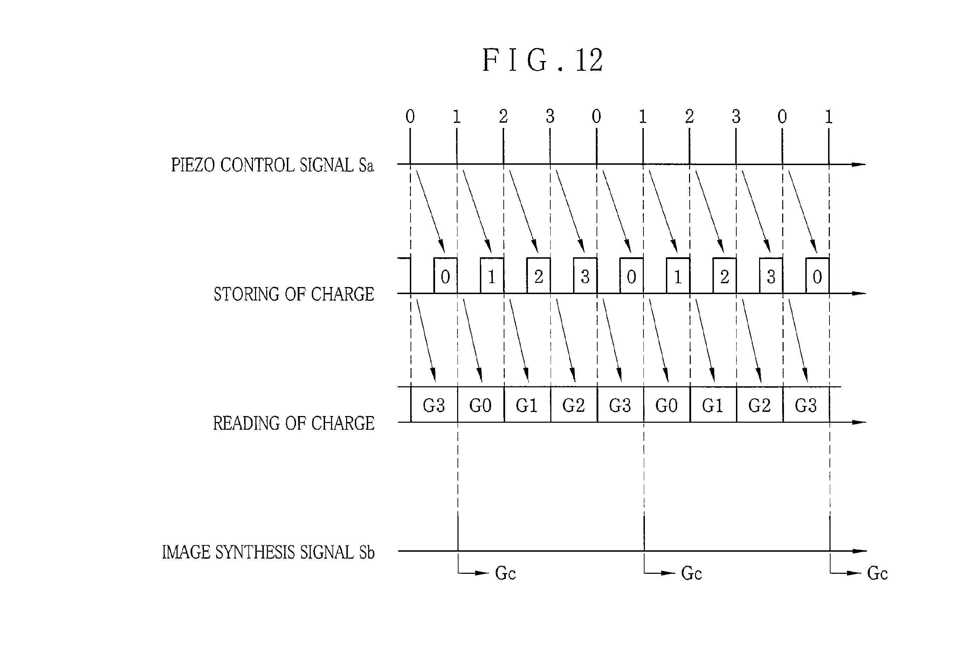

In Fig. 12, a mode of the four shift events is illustrated. Immediately after completing storing of the charge in the CCD group 58, the sync control unit 62a generates a piezoelectric control signal Sa. This is when the signal charge of one frame is read to a vertical transfer path from the pixels 81 of the CCD group 58 (or when a reading pulse is output by the CCD driver 60 to the CCD group 58). Also, the sync control unit 62a generates an image synthesis signal Sb upon completion of reading the charge of the CCD group 58 in correspondence with the image G3 obtained at the third set position. The operation of reading the charge is a sequence of CCD operation inclusive of reading the signal charge from the pixels 81 of the CCD group 58 to the vertical path, and vertical transfer, horizontal transfer and an output of an image signal of one frame.

The piezoelectric driver 61 in response to the piezoelectric control signal Sa supplies the piezoelectric actuator material 35 with a predetermined voltage, to displace the shift mechanism 38 from a previous set position to a present set position. Shift time from an output of the piezoelectric control signal Sa from the sync control unit 62a to the piezoelectric driver 61 until shift of the shift mechanism 38 to a succeeding set position is shorter than clearing time from completion of previous storing of charge in the CCD group 58 until a start of succeeding storing of charge. Thus, succeeding storing of charge is always started while the shift mechanism 38 is kept set in the succeeding set position by the piezoelectric driver 61.

The image synthesizing unit 65a in response to the image synthesis signal Sb reads images G0-G3 obtained from the set positions of displacement. The image synthesizing unit 65a maps pixels of the images G0-G3 according to the set positions of displacement, and outputs a synthesized image Gc. It is further possible to interpolate the pixels to process the images G0-G3 or synthesized image Gc in the course of the synthesis.

In the synthesized image Gc, a loss region due to the cladding 51 in transmitting image light can be compensated for in a visible manner. Pixel values of the pixels of the portions are directly derived from the object image without approximation or interpolation of adjacent pixels within one frame. Consequently, the number of pixels is higher than that in images obtained from the normal imaging mode or according to each one of the set positions of displacement, to produce the image in a very fine quality. Note that the image quality is higher in images obtained according to the nine shift events than images of the four shift events.

It is to be noted that the images G0-G3 are different part images 80 with differences in the set position by displacement. The part image 80 at the distal tip 48 is only shifted by keeping a proximal tip of the fiber optic image guide 31 stationary. No change occurs in the relative position between the proximal tip of the fiber optic image guide 31 and an image pickup surface of the CCD group 58. There are no apparently distinct features between data output according to the pixels 81 even with the various set positions. For example, the part image 80 of a position in the image G0 is different from the part image 80 of the same position in the image G1 in relation to the set position. However, those are recorded commonly by the pixels 81 on the CCD group 58. Accordingly, the image synthesizing unit 65a determines original pixels of pixel values of the images among the pixels 81 by mapping on the basis of the relative position of the part image 80 of the displacement information 84 and the pixels 81, to carry out the pixel interpolation.

The operation of the endoscope system 2 of the above construction is described now. To observe a body part of a patient endoscopically, a doctor or operator connects the endoscope 5 to the processing apparatus 6 and the light source apparatus 7, which are turned on and powered. The input interface 68 is manually operated to input information related to the patient, to start examination.

After instructing the start, the doctor or operator enters the elongated tube 9 in the body. Light from the light source apparatus 7 is applied to body parts, while he or she observes an image on the display panel 17 from the CCD group 58 of the image sensor.

An image signal is generated by the CCD group 58, processed by the analog front end 59 for various functions of processing, and input to the digital signal processor 65. The digital signal processor 65 processes the image signal for various functions of signal processing, to produce an image. The image from the digital signal processor 65 is output to the digital image processor 66.

The digital image processor 66 is controlled by the CPU 62 and processes the image from the digital signal processor 65 for various functions of image processing. The image is input by the digital image processor 66 to the display control unit 67. According to the graphic data from the CPU 62, the display control unit 67 carries out control for display. Thus, the image is displayed on the display panel 17 as an endoscopic image.

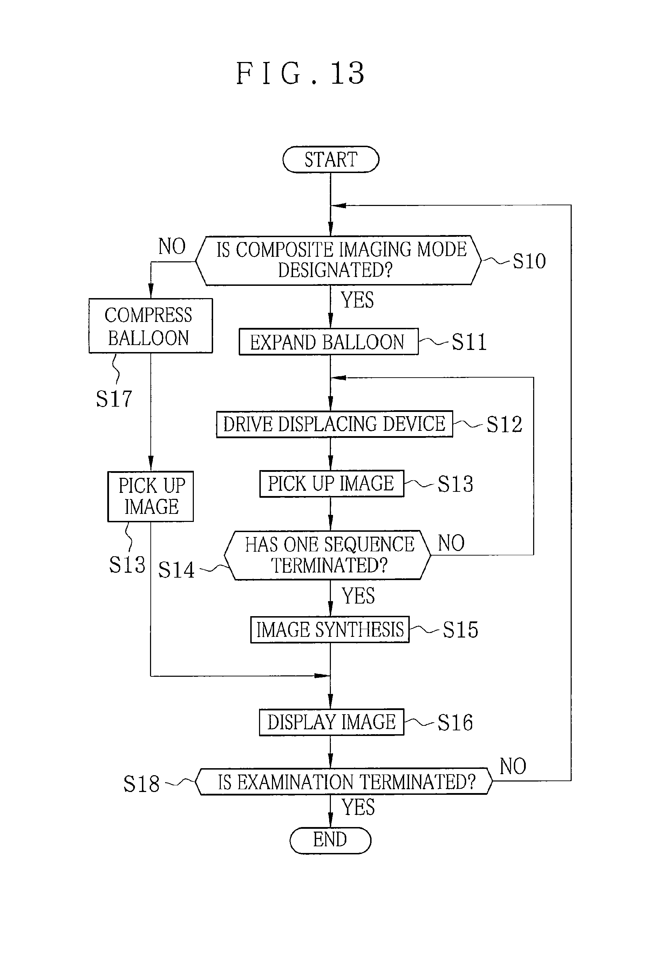

In Fig. 13, the composite imaging mode is selected (yes in the step S10). The CPU 62 of the processing apparatus 6 sends the expansion signal to the pump driver 83 of the pump apparatus 8. The pump driver 83 drives the rotary pump 82 to charge water from the tank 24 to the balloon 18 at a predetermined amount to expand the balloon 18 in the step S11. The balloon 18 tightly contacts a wall of a body cavity to anchor the head assembly 16 for stabilization.

Then the sync control unit 62a and the piezoelectric control unit 62b are ready in the CPU 62 in the processing apparatus 6. According to the displacement information 84 and information of drive pulses from the CCD driver 60 for the CCD group 58, the sync control unit 62a sends the piezoelectric control signal Sa to the piezoelectric control unit 62b, and sends the image synthesis signal Sb to the image synthesizing unit 65a.

The operation of the piezoelectric driver 61 is controlled by the piezoelectric control unit 62b upon receiving the piezoelectric control signal Sa. The piezoelectric driver 61 applies a predetermined voltage to the piezoelectric actuator material 35. Thus, the shift mechanism 38 displaces at a predetermined angle and pitch according to the designated number of the shift events. See the step S12. At each time that the shift mechanism 38 is retained in one of the set positions, charge is stored in the CCD group 58. The part image 80 of a body part is picked up by the pixels 81 through the fiber optic image guide 31 in the step S13. A sequence including the steps S12 and S13 is repeated (no at the step S14) until the end of one two-dimensional shift sequence by shift of the shift mechanism 38 from the initial position and again to the same position.

When one two-dimensional shift sequence is terminated (yes at the step S14), image synthesis is carried out by the image synthesizing unit 65a upon receiving the image synthesis signal Sb, to produce a synthesized image at the step S15 from the images obtained according to the set positions of fine displacement. After data of the synthesized image is processed in the digital image processor 66 and the display control unit 67, the synthesized image is displayed on the display panel 17 at the step S16. In contrast, when the normal imaging mode is designated, the pump driver 83 drives the rotary pump 82 upon receiving a compression signal as command signal, to return water from the balloon 18 to the tank 24. The balloon 18 is compressed in the step S17. Then an image is picked up in the step S13 but without carrying out the sequence of the steps S12 and S15. Those steps are repeated until a termination signal for terminating the examination as command signal is input (yes at the step S18). If the balloon 18 has been expanded upon receiving the termination signal, the task of the step S17 is performed to compress the balloon 18 to terminate the examination.

As described heretofore, the balloon 18 is secured to the elongated tube 9 of the endoscope 5 and expanded to anchor the head assembly 16 in a body cavity. This is effective in stabilizing the head assembly 16 by preventing vibration with influence of displacement of the distal tip 48. An image of high quality can be obtained endoscopically without shake.

As the balloon 18 is expanded automatically upon selection of the composite imaging mode in a selectable setting of the normal imaging mode and the composite imaging mode, it is possible not to add a start switch for expanding the balloon 18.

It is possible selectively to set the normal imaging mode and the composite imaging mode, so as to reflect the intention of the doctor or operator. When the elongated tube 9 of the endoscope 10 is entered in the body and advanced, the normal imaging mode is sufficient, because a motion image can be created smoothly in a continuous manner without delay of time relative to motion of an object even with a low image quality. As the elongated tube 9 moves, it is unnecessary to expand the balloon 18.

In contrast with this, the composite imaging mode is effectively designated for detailed imaging after the reach of the distal end of the elongated tube 9 to an object of interest with affected tissue or the like, so that an image suitable for diagnosis can be provided because of its higher image quality than the normal imaging mode. As the elongated tube 9 is located short of the object of interest, it is necessary to anchor the head assembly 16 on a wall of a body cavity by expanding the balloon 18. Thus, it is possible to use the elongated tube 9 suitably without complicated handling by compressing the balloon 18 in the normal imaging mode and by expanding the balloon 18 in the composite imaging mode.

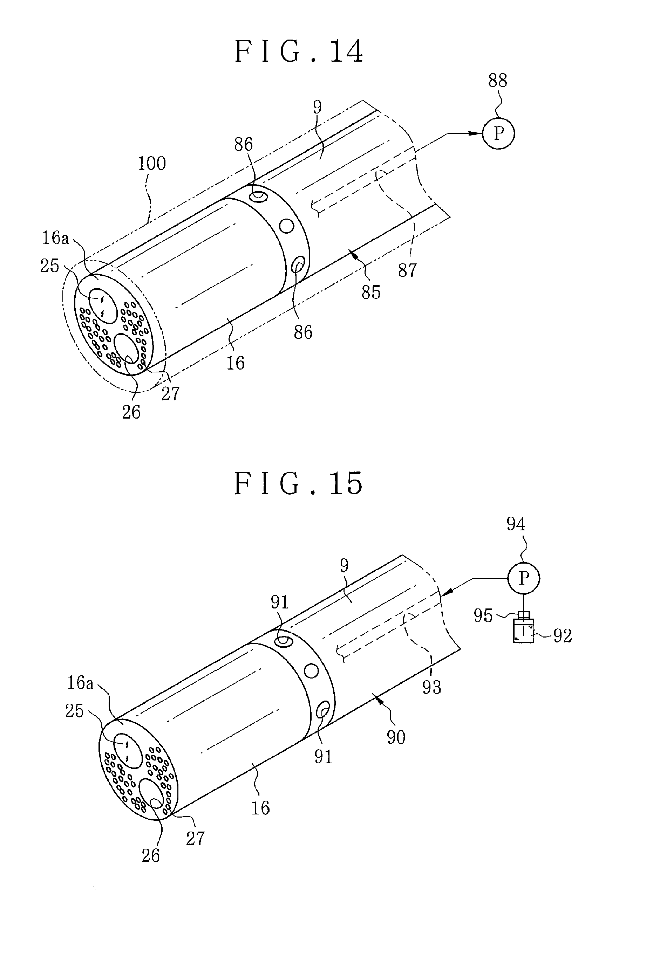

Various anchoring devices can be used for anchoring the head assembly 16 in place of the balloon 18 and the rotary pump 82 of the above. In Fig. 14, one preferred endoscope 85 is illustrated. Suction holes 86 as an anchoring device are formed along the borderline between the elongated tube 9 and the head assembly 16. A negative pressure is generated through the suction holes 86 to suck a wall of a body cavity to anchor the head assembly 16 for stabilization.

In Fig. 14, the suction holes 86 are arranged equidistantly (for example, at an interval of 45 degrees) about the elongated tube 9. The suction holes 86 are eight holes. There is a suction channel 87 of which a first end is open at the suction holes 86 in a manner similar to the fluid port 29a. The suction channel 87 extends through the elongated tube 9 to the connection opening 19. A tube (not shown) is coupled with the connection opening 19. A suction pump 88 is connected by the tube with the suction channel 87, and operates for generating a negative pressure in the suction holes 86.

When the composite imaging mode is selected, the suction pump 88 is driven to generate a negative pressure in the suction holes 86. A wall located about the suction holes 86 is sucked, to anchor the head assembly 16 in the body cavity.

In Fig. 15, another preferred endoscope 90 is illustrated. Ejection nozzles 91 as an anchoring device are formed along the borderline between the elongated tube 9 and the head assembly 16 in a manner similar to the suction holes 86. Anchoring agent 92 or adhesive agent is ejected through the ejection nozzles 91. Examples of the anchoring agent 92 include glycerine and the like having biocompatibility and suitable viscosity. Furthermore, the anchoring agent 92 may be a material with small adhesion property, and a material hardenable upon reaction with water in the body cavity.

There is a delivery channel 93 similar to the fluid channel 29 and the suction channel 87. The ejection nozzles 91 are open at one end of the delivery channel 93. A tube (not shown) is coupled with the connection opening 19. A delivery pump 94 for the anchoring agent is connected with the delivery channel 93 by the connection opening 19 and the tube. A bottle 95 or reservoir stores the anchoring agent 92. When the composite imaging mode is selected, the delivery pump 94 is driven to draw the anchoring agent 92 from the bottle 95, and causes ejection of the anchoring agent 92 through the ejection nozzles 91 upon passage through the delivery channel 93. In conclusion, effects similar to the above embodiments can be obtained in the construction of Figs. 14 and 15.

The number and disposition of the suction holes 86 and the ejection nozzles 91 are not limited to the above examples. The suction holes 86 and the ejection nozzles 91 may be arranged randomly, or regularly in a direction aligned with the axial direction or peripheral direction of the elongated tube 9. Also, the suction holes 86 and the ejection nozzles 91 may be arranged spirally, or eccentrically, for example in a lower area as viewed in Fig. 2. In the above embodiments, the anchoring device operates automatically when the composite imaging mode is selected. However, a specific switch may be added and operated manually for actuating the anchoring device.

In the above embodiments, each anchoring device is associated with the endoscope. However, a sleeve device 100 or barrel of Fig. 14 can be used for covering the elongated tube of the endoscope, and an anchoring device can be associated with the sleeve device 100. A doctor or operator handles the elongated tube by entry of the sleeve device 100 together with the elongated tube. The sleeve device 100 has an inner bore slightly greater than an outer diameter of the elongated tube, and is disposed about the elongated tube to dispose the balloon, suction holes or ejection nozzles near to the head assembly. This is effective in protecting the elongated tube as the endoscope can be unchanged without modification.

In relation to the balloon 18 as anchoring device, it is possible suitably to determine a tube portion of the elongated tube 9 to anchor on a wall of a body cavity. In short, the balloon 18 can anchor a tube portion of the elongated tube 9 located around the 30, a tube portion located around the distal tip 48, or a tube portion located around the piezoelectric actuator material 35.

A displacing device may be shaped in a form other than the rod form, for example, in a form of a quadrilateral prism. A fiber optic image guide is inserted in and attached to a support casing by an adhesive agent (not shown) or the like. A piezoelectric actuator material is overlaid on four faces of the support casing by way of electrodes. The fiber optic image guide is shifted together with the support casing up and down and to the right and left. For example, the displacing device displaces from the initial position in the leftward direction by 90 degrees at an amount of (3^{1/2}) /4.P to come to the set position with a first shift event. After this, the displacing device is returned to the initial position, and displaces in the downward direction at an amount of 1/4.P to come to the set position with a second shift event. The displacing device is returned from the set position of the second shift event to the initial position, and displaces to the right and upwards, and then is returned to the initial position again. Thus, the core 50 displaces in the two-dimensional path of a crossed shape by fine displacement. For the calibration, two calibration charts can be prepared for vertical and horizontal directions. A width of each of black and white areas can be set a constant value times the shift amount in the vertical and horizontal directions from an initial position.

However, there is a characteristic of the hysteresis in the piezoelectric actuator materials, of which the set position may be offset upon driving the piezoelectric actuator materials without a patterned manner. Thus, the displacing device is caused to displace with the same two-dimensional path and in the same sequence. In short, a sequence of driving the piezoelectric actuator materials for actuating the displacing device is set equal for every event. Also, a sequence of supplying electrodes with voltage on a couple of the right and left sides is kept the same.

As the fiber optic image guide is displaced by tilt of a root portion of the shift mechanism, the fiber optic image guide is likely to vibrate and stop with lag in the set positions without immediate stop. Thus, it is preferable with a piezoelectric driver to drive the piezoelectric actuator material or to use other anti-vibration methods in order to tilt the shift mechanism instantaneously in reverse to the displacing after a stop of the displacing device. Specifically, reaction force is previously measured by simulation or experimentally. An offset voltage for the piezoelectric actuator material is stored in a ROM. The piezoelectric control unit reads the information of the offset voltage from the ROM and sets the information in the piezoelectric driver. Furthermore, non-conductive fluid with high viscosity can be charged in a lumen for an anti-vibration structure by utilizing damping effect.

In the above embodiment, shift time for the shift mechanism to displace to a succeeding position is shorter than clearing time from previous completion of storing charge of the CCD until a succeeding start of storing charge. However, the shift time may be longer than the clearing time for the reason of various factors, which include a length, material or shift amount of the shift mechanism, performance of the piezoelectric actuator material, or the like. In considering that the weight of the fiber optic image guide is relatively large, the shift time is very likely to be longer than the clearing time.

While the shift mechanism becomes set in the set position, the CCD driver is controlled by the CPU of the processing apparatus and supplies the CCD with an electronic shutter pulse. A start of storing charge is delayed. When the shift mechanism stops in the set position, storing charge is started. Otherwise, the light source is turned off while the shift mechanism becomes set in the set position, and then turned on when the shift mechanism stops in the set position.

In order to drive the CCD with reference to time required for shift to a succeeding one of the set positions, the frame rate must be set small should the shift time be longer than the clearing time. However, it is possible without blur to obtain an image even with the presently high frame rate by sweeping out charge with an electronic shutter pulse, or by turning off the light source, or by other methods.

In the above embodiment, the image synthesizing unit synthesizes the image only when the composite imaging mode is designated. However, it is possible to synthesize an image also in the normal imaging mode. This is effective in compensating for a loss region of the cladding even though an image for reflecting an object image positioned in association with the cladding cannot be obtained. Image quality can be high.

In the above embodiment, the image synthesizing unit carries out synthesis for one two-dimensional shift sequence, to output one synthesized image. However, a problem occurs in that a frame rate of the composite imaging mode is lower than the normal imaging mode. To solve this problem, it is preferable to raise a frame rate to four times as high a level as the frame rate for the normal imaging mode, namely change over the frame rate in response to designating the composite imaging mode.

Specifically, frequency of the clock signal of the system clock in the CPU 62 is changed to change frequency of a drive signal of the CCD driver 60. Otherwise, it is possible in the CCD driver 60 to dispose a clock divider without changing the clock signal. The clock signal can be divided by the clock divider to change the frequency.

In another variant of the embodiment of the four shift events, the images G0-G3 of one two-dimensional shift sequence is used to create a synthesized image Gc. Then the images G0-G3 and one image G0 according to a second two-dimensional shift sequence can be used to create a synthesized image Gc. Similarly, a combination of images G0-G3 for one two-dimensional shift sequence can be changed by one image. An earliest one of the images G0-G3 is replaced with a newest one of images G0-G3 so as to create a series of synthesized images Gc. This is effective in preventing complexity in the control by changing a period of a clock signal. A drop of the frame rate can be prevented.

Elements of the hardware can be incorporated in a housing separate from the processing apparatus, or may be incorporated in the endoscope, the hardware including the three-CCD assembly, the input interface for setting the imaging mode and the number of shift events, and electronic circuits to constitute the image synthesizing unit, sync control unit and piezoelectric control unit.

One preferred light source apparatus can include a blue laser light source, which may have a characteristic with a central wavelength of 445 nm. A wavelength conversion device is disposed on an exit side of the light guide devices 27 at its distal end. The wavelength conversion device contains plural phosphors which partially absorb the laser light from the blue laser light source, to emit converted light with colors from green to yellow by excitation. The laser light from the blue laser light source and the excitation light from green to yellow after the conversion are coupled together, to produce white light of high brightness. It is possible to obtain sufficiently bright light with a small number of light guide devices, namely one or two, because white light can be delivered with higher brightness than the former embodiment. The diameter of the endoscope can be reduced more effectively for an ultra thin tube.

Note that a single CCD assembly may be used instead of a three-CCD assembly. In the above embodiments, the first connector 11 is used commonly for the connection of the fiber optic image guide and the connection cable to the processing apparatus. However, two separate connectors may be used and may contain the fiber optic image guide and the connection cable discretely.

In the above embodiment, the CCD group 58 is contained in the processing apparatus 6. However, an endoscope of the invention may contain the CCD group 58. The processing apparatus 6 can be supplied with image signals from the CCD group 58.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope for imaging of an object in a body cavity by receiving image light on an image sensor (56), comprising:
an elongated tube (9) for entry in said body cavity;
an objective lens system (30), incorporated in said elongated tube, for receiving said image light from said object;
a fiber optic image guide (31), including a plurality of bundled optical fibers (52) and a distal tip (48), inserted in said elongated tube, for transmitting said image light focused on said distal tip by said objective lens system;
a piezoelectric actuator (35), positioned on a periphery of said distal tip of said fiber optic image guide, for displacing said distal tip periodically, wherein said image sensor detects said image light from said fiber optic image guide for plural times in synchronism with said displacement for forming a synthesized image;
an anchoring device (18, 86, 91) for anchoring said elongated tube partially on a wall of said body cavity, to stabilize a position of said elongated tube relative to said image light incident thereon.

2. An endoscope as defined in claim 1, wherein said anchoring device includes a resilient balloon, secured to a peripheral surface of said elongated tube removably in an air tight manner, for expanding by delivery of fluid with a pump to push said wall of said body cavity.

3. An endoscope as defined in claim 2, further comprising a fluid channel, formed through said elongated tube, for delivering said fluid between said balloon and said pump.

4. An endoscope as defined in claim 1, further comprising a sleeve device disposed about said elongated tube;
wherein said anchoring device is positioned on a peripheral surface of said sleeve device.

5. An endoscope as defined in claim 1, wherein said anchoring device includes a suction hole, formed in a peripheral surface of said elongated tube, for sucking said wall of said body cavity by generating a negative pressure with a suction pump.

6. An endoscope as defined in claim 5, further comprising a suction channel, formed through said elongated tube, for drawing air between said suction hole and said suction pump.

7. An endoscope as defined in claim 1, wherein said anchoring device includes an ejection nozzle, formed in a peripheral surface of said elongated tube, for ejecting anchoring agent delivered by a delivery pump.

8. An endoscope as defined in claim 7, further comprising a delivery channel, formed through said elongated tube, for delivering said anchoring agent from said delivery pump to said ejection nozzle.

9. An endoscope as defined in claim 1, wherein said anchoring device is actuated in response to a signal input with an input interface.

10. An endoscope as defined in claim 9, wherein said input interface determines whether said distal tip should be displaced to generate a displacement signal;
if said input interface generates said displacement signal, then said anchoring device is automatically actuated.
